# EUROPEAN PATENT APPLICATION

(11) **EP 2 656 779 A1**
(43) Date of publication of application: **30.10.2013**
(21) Application number: 12832778.0
(22) Date of filing: 14.11.2012
(51) Int. Cl.: A61B 1/06, G02B 23/24

(54) **ENDOSCOPE**

(30) Priority: 31.01.2012 JP 2012018783
(71) Applicant: OLYMPUS MEDICAL SYSTEMS CORP., Tokyo 151-0072 (JP)
(72) Inventor: SAKATA, Hajime, Tokyo 151-0072 (JP)
(74) Representative: Gunzelmann, Rainer
(86) International application number: PCT/JP2012/079459
(87) International publication number: WO 2013/114703

(57) **Abstract**

An endoscope includes an endoscope connector at a proximal end portion of a universal cable that is extended from an endoscope operation portion. The endoscope connector includes: a first unit that is provided at the proximal end portion of the universal cable, and inside which are inserted a fluid conduit and a signal transmission line that are inserted through inside of the universal cable; a second unit that is connected and fixed to the first unit, and that internally includes a connection conduit that is connected to the fluid conduit; and a signal transmission cable that is integrated with a side portion of the first unit and includes an electrical connector at an end portion.

## Description

### Technical Field

The present invention relates to an endoscope that has an endoscope connector that is integrally equipped with a scope cable at an end portion of a universal cable.

### Background Art

Some endoscopes have an endoscope connector that is integrally equipped with a scope cable at an end portion of a universal cable that extends from an operation portion of the endoscope.

In Japanese Patent Application Laid-Open Publication No. 2001-252245, an endoscope is disclosed that is mainly constituted by an insertion portion, an operation portion that is arranged at a proximal end portion of the insertion portion, a universal cable that extends from, for example, a proximal end face of the operation portion, and a light guide connector (corresponds to an endoscope connector of the present invention) that is provided at a proximal end portion of the universal cable. A camera cable corrugated tube (corresponds to a signal transmission cable of the present invention) that is a scope cable branches from a side portion of the light guide connector of the endoscope. A camera connector (corresponds to an electrical connector of the present invention) that is connected to a video processor is provided at a proximal end portion of the camera cable corrugated tube.

In the endoscope disclosed in Japanese Patent Application Laid-Open Publication No. 2001-252245, an observation window, an illuminating window, a nozzle, and a suction port and the like are provided at a distal end portion of the insertion portion. An image pickup unit in which an image pickup surface of an image pickup device is disposed on an optical axis of the observation window is provided inside the distal end portion.

A first signal transmission line that transmits image pickup signals is connected to the image pickup unit. A distal end face of a light guide fiber bundle that transmits illuminating light is arranged on a proximal end face side of the illuminating window. A distal end portion of an air feeding tube that supplies air, for example, and a distal end portion of a liquid feeding tube that supplies water, for example, are provided in the nozzle. A distal end portion of a suction tube is provided in the suction port.

The first signal transmission line, the light guide fiber bundle, the suction tube, the air feeding tube, and the liquid feeding tube are inserted through the inside of the insertion portion, the operation portion, and the universal cable and extend into the endoscope connector. The first signal transmission line further extends into the camera connector via the inside of the camera cable corrugated tube that is integrated with a side portion on the proximal end side of the endoscope connector. The camera connector is detachably attachable to a connector of a video processor that is an external device.

Various switches are provided on the operation portion. The switches are connected to a second signal transmission line that transmits switching signals. The second signal transmission line extends into the camera connector through the inside of the operation portion, the universal cable, the endoscope connector, and the camera cable corrugated tube.

Accordingly, with the above described endoscope assembling, work is required to insert tubes through the inside of the endoscope connector and work is also required to attach the tubes. In addition, work is required to insert the two kinds of signal transmission lines through the inside of the endoscope connector. Thus, operations of the work have been burdensome for a worker. On the other hand, when a blockage or the like has arisen in a tube, a worker performs work to replace the tube. In this case, in order to prevent a trouble of breaking the signal transmission line from occurring in the work to replace the tube, the worker cancels the connection of the signal transmission line in the camera connector in advance and withdraws the transmission cable from the endoscope connector and then performs the work to replace the tube. Therefore, it takes a long time to perform the work to replace the tube.

The present invention has been made in view of the above circumstances and an object of the present invention is to provide an endoscope capable of easily performing assembly work or repair work, etc. of an endoscope connector which is integrally equipped with a scope cable.

### Disclosure of Invention

### Means for Solving the Problem

An endoscope according to one aspect of the present invention includes an endoscope connector at a proximal end portion of a universal cable that is extended from an endoscope operation portion, in which the endoscope connector includes: a first unit that is provided at the proximal end portion of the universal cable, and inside which are inserted a fluid conduit and a signal transmission line that are inserted through inside of the universal cable; a second unit that is connected and fixed to the first unit, and that internally includes a connection conduit that is connected to the fluid conduit; and a signal transmission cable that is integrated with a circumferential portion of the first unit and includes an electrical connector at an end portion.

### Brief Description of the Drawings

Fig. 1 is a view that illustrates an endoscope apparatus that includes an endoscope and an outside apparatus;
Fig. 2 is a view that illustrates an endoscope connector into which a signal transmission cable is integrated;
Fig. 3 is a schematic diagram that illustrates a relation between various fluid conduits and various signal transmission lines that are inserted through the inside of the endoscope, and the endoscope connector;
Fig. 4 is a view that illustrates a state in which the endoscope connector has been separated into a first unit and a second unit;
Fig. 5 is a view that illustrates a first case body;
Fig. 6 is a view that illustrates an endoscope that includes an insertion portion, an operation portion, a universal cable, and an endoscope connector, and inner space of the endoscope;
Fig. 7 is a view that illustrates a first region at which a water immersion detecting seal is affixed;
Fig. 8 is a view that illustrates a second region at which a water immersion detecting seal is affixed;
Fig. 9 is a view that illustrates a third region at which a water immersion detecting seal is affixed;
Fig. 10 is a view that illustrates a water leakage detecting pipe sleeve that has a check valve;
Fig. 11 is a view that illustrates a pipe sleeve inner face region in the vicinity of a water leakage detecting pipe sleeve mounting portion at which a water immersion detecting seal is affixed;
Fig. 12 is a view that shows an endoscope connector in which a light guide pipe sleeve and an air feeding pipe sleeve are adjacent;
Fig. 13 is a perspective view that illustrates an endoscope connector in which a light guide pipe sleeve and an air feeding pipe sleeve are provided in a pipe sleeve holding member;
Fig. 14 is a longitudinal cross-sectional view that illustrates the endoscope connector in which the light guide pipe sleeve and the air feeding pipe sleeve are provided in the pipe sleeve holding member;
Fig. 15 is a view that illustrates a water leakage detecting pipe sleeve of an endoscope connector, and an air pipe sleeve that is connected to the water leakage detecting pipe sleeve and supplies air;
Fig. 16 is a view that illustrates a state in which a cam groove of the air pipe sleeve is disposed on a cam pin of the water leakage detecting pipe sleeve;
Fig. 17 is a cross-sectional view of the water leakage detecting pipe sleeve;
Fig. 18A is a cross-sectional view of a water leakage detecting pipe sleeve that has a characteristic cam pin;
Fig. 18B is a cross-sectional view along a line indicated by arrows Y18B-Y18B in Fig. 18A;
Fig. 19 is a view that illustrates a universal cable proximal end portion, an endoscope connector, a bend preventing element, and inner space of an endoscope that constitute an endoscope;
Fig. 20 is a view that illustrates a cable flexible portion of a cable constituent member;
Fig. 21 is a view that illustrates a state in which a first pipe sleeve is adhesively fixed to the cable flexible portion;
Fig. 22 is a view that illustrates a state in which a second pipe sleeve is adhesively fixed to the first pipe sleeve; and
Fig. 23 is a view that illustrates a state in which a connector framework part is fastened with screws to the second pipe sleeve.

### Best Mode for Carrying Out the Invention

Embodiments of the present invention are described hereunder with reference to the drawings.

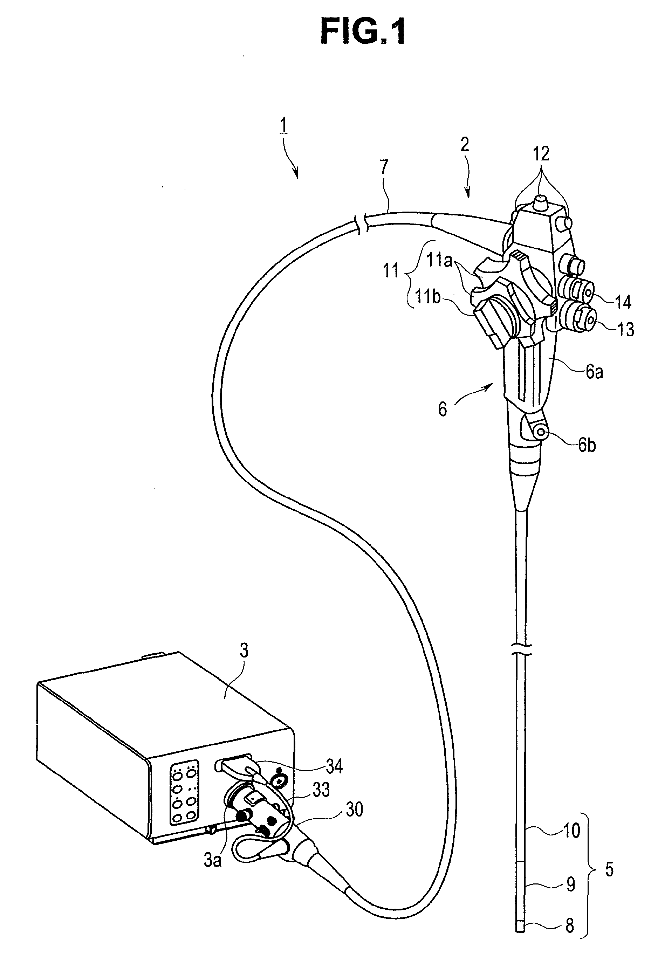

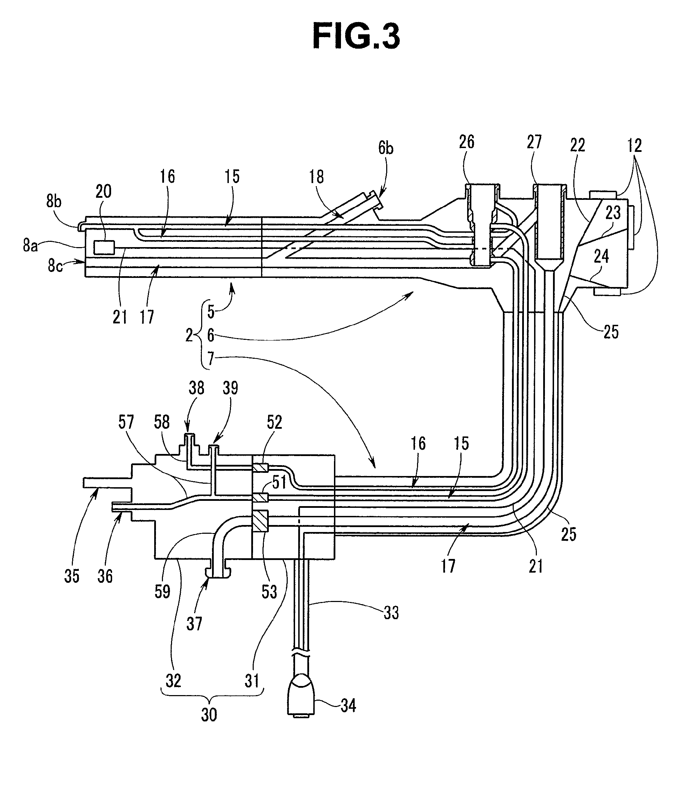

As shown in Fig. 1, an endoscope apparatus 1 includes an endoscope 2 and, for example, a camera control unit (hereunder, referred to as "CCU") 3 and a monitor (not shown in the drawings) that are outside apparatuses. The CCU 3 of the present embodiment serves as both a light source apparatus and a video processor. That is, the CCU 3 incorporates a light source for supplying illuminating light to the endoscope 2, and a signal processing circuit that performs various kinds of signal processing on signals from an image pickup device that the endoscope 2 includes and the like. An air feeding pump (not shown) that supplies air or the like to an air feeding conduit (see reference numeral 15 in Fig. 3) that is described later is also provided inside the CCU 3.

The endoscope 2 includes an insertion portion 5, an operation portion 6, and a universal cable 7. The insertion portion 5 is an elongated long member that is inserted into a site to be observed. The insertion portion 5 includes a distal end portion 8, a bending portion 9, and a flexible tube portion 10 that are connected in series. An illumination optical system that includes a light guide (not shown) and an image pickup optical system that includes an image pickup apparatus (denoted by reference numeral 20 in Fig. 3) are contained inside the distal end portion 8. A nozzle (see reference symbol 8b in Fig. 3) and a suction port (see reference symbol 8c in Fig. 3) that also serves as a treatment instrument lead-out port are provided in a distal end face (see reference symbol 8a in Fig. 3) of the distal end portion 8. The bending portion 9 is configured to be bendable in, for example, four directions, namely the upward, downward, left and right directions. The flexible tube portion 10 is a tube-shaped member that is long and has flexibility.

The operation portion 6 includes a grasping portion 6a. The grasping portion 6a is connected in series to a proximal end portion of the insertion portion 5. A bending operation portion 11, various switches 12, an air/water feeding button 13, and a suction button 14 and the like are provided on the operation portion 6. The bending operation portion 11 has a bending operation knob 11a and a fixing lever 11b. The bending operation knob 11 a is a knob for performing a bending operation of the bending portion 9. The fixing lever 11b is a lever for fixing the bending operation knob 11a at a desired rotational position. The switches 12 are, for example, a release switch, a freeze switch, and an observation mode switching switch for switching between normal observation and fluorescence observation or the like. Reference symbol 6b denotes a treatment instrument insertion port.

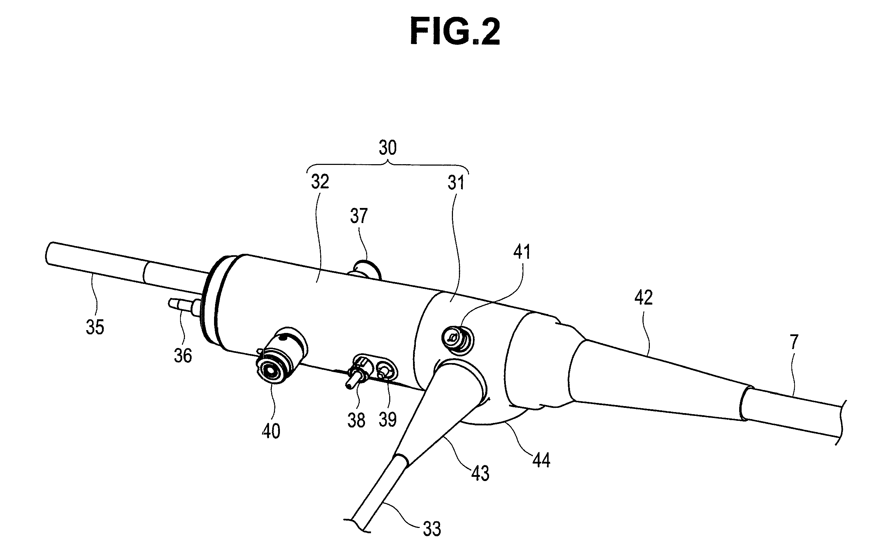

The universal cable 7 extends from a side of the operation portion 6. An endoscope connector 30 shown in Fig. 2 is provided at an end portion of the universal cable 7.

As shown in Fig. 2 and Fig. 3, the endoscope connector 30 of the present embodiment includes a first unit 31 that constitutes a distal end side and a second unit 32 that constitutes a proximal end side. The endoscope connector 30 is constituted by connecting the first unit 31 and the second unit 32.

The first unit 31 is integrally provided at a proximal end portion of the universal cable 7. A signal transmission cable 33 that is a scope cable is integrally provided at a side portion of the first unit 31. An electrical connector 34 is provided on the other end side of the signal transmission cable 33. The other end of the signal transmission cable 33 is connected using means such as soldering or a micro-connector to a substrate provided inside the electrical connector 34.

A light guide pipe sleeve 35 and an air feeding pipe sleeve 36 that is an external connection portion are provided in a protruding manner from a proximal end face of the second unit 32. In addition, a suction pipe sleeve 37, a water feeding pipe sleeve 38, and a pressurization pipe sleeve 39 that are external connection portions are provided in a protruding manner from a side portion of the second unit 32.

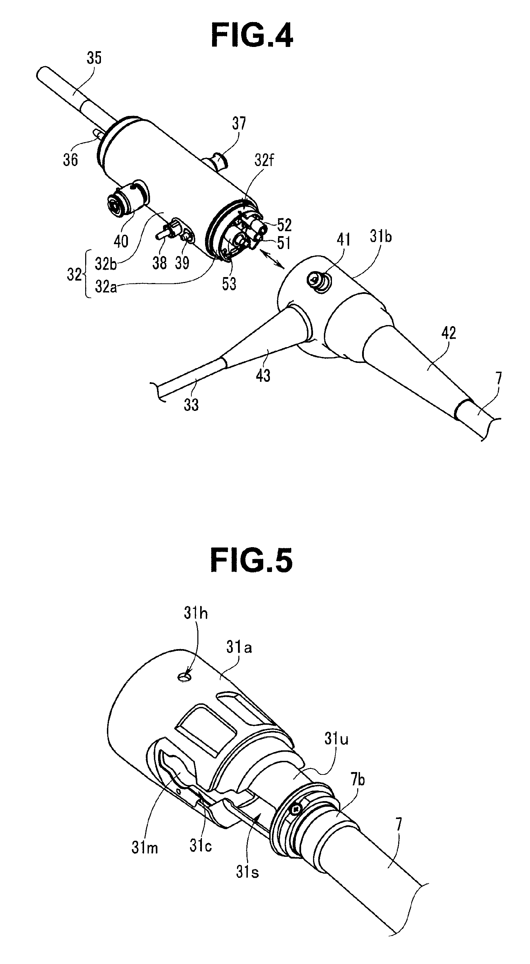

Note that reference numeral 40 denotes a water leakage detecting pipe sleeve, reference numeral 41 denotes an earth terminal, reference numeral 42 denotes a universal cable bend preventing element, reference numeral 43 denotes a signal transmission cable bend preventing element, and reference numeral 44 denotes a convex portion with a built-in tag. The earth terminal 41 is mounted in a conductive state with respect to a first case body that is described later (see reference symbol 3 1 a in Fig. 5). The universal cable bend preventing element 42 and the signal transmission cable bend preventing element 43 are cover members that protect connection portions. The universal cable bend preventing element 42 prevents buckling and the like of the universal cable 7 and the signal transmission cable 33. The signal transmission cable bend preventing element 43 prevents buckling and the like of the signal transmission cable 33. An RFID chip as a solid body identification portion is contained inside the convex portion with a built-in tag 44.

As shown in Fig. 3, an image pickup cable 21 that is a first signal transmission line extends from the image pickup apparatus 20. The image pickup cable 21 extends into the electrical connector 34 through the inside of the insertion portion 5, the operation portion 6, the universal cable 7, the first unit 31, and the signal transmission cable 33.

Signal lines 22, 23, and 24 that are each a second signal transmission line extend from the various switches 12. The signal lines 22, 23, and 24 are bundled as a signal cable 25, and the signal cable extends into the electrical connector 34 through the inside of the operation portion 6, the universal cable 7, the first unit 31, and the signal transmission cable 33. It is not a particular problem even if the signal lines 22, 23, and 24 are not bundled.

The nozzle 8b is configured to communicate with one end side of the air feeding conduit 15 that is a fluid conduit and one end side of the water feeding conduit 16 that is a fluid conduit. The air feeding conduit 15 and the water feeding conduit 16 are each inserted through the inside of the insertion portion 5, the operation portion 6, and the universal cable 7 and extend inside the first unit 31.

The other end of the air feeding conduit 15 is connected to an air feeding connector 51 that is a connection portion provided on a distal end face side of the second unit 32. The other end of the water feeding conduit 16 is connected to a water feeding connector 52 that is a connection portion provided on the distal end face side of the second unit 32.

One end side of a suction conduit 17 that is a fluid conduit communicates with the suction port 8c. The suction conduit 17 extends into the first unit 31 through the inside of the insertion portion 5, the operation portion 6, and the universal cable 7. The other end of the suction conduit 17 is connected to a suction connector 53 that is a connection portion provided on the distal end face side of the second unit 32.

As shown in Fig. 3 and Fig. 4, the air feeding connector 51, the water feeding connector 52, and the suction connector 53 that are the aforementioned connection portions are provided on the distal end face side of the second unit 32.

The air feeding connector 51 communicates with the air feeding pipe sleeve 36 and the pressurization pipe sleeve 39 by means of an in-unit air feeding conduit 57 that is a connection conduit provided inside the second unit. The water feeding connector 52 communicates with the water feeding pipe sleeve 38 by means of an in-unit water feeding conduit 58 that is a connection conduit. The suction connector 53 communicates with the suction pipe sleeve 37 by means of an in-unit suction conduit 59 that is a connection conduit.

Note that the in-unit air feeding conduit 57, the in-unit water feeding conduit 58, and the in-unit suction conduit 59 are constituted by a flexible tube, a rigid tube, or a hole formed in a block or the like.

In addition, a light guide that is not shown in the drawings extends into the light guide pipe sleeve 35 through the inside of the insertion portion 5, the operation portion 6, the universal cable 7, the first unit 31, and the second unit 32. Reference numeral 18 denotes a treatment instrument conduit. One end side of the treatment instrument conduit 18 communicates with the treatment instrument insertion port 6b, and the other end side thereof communicates with an intermediate portion of the suction conduit 17. Reference numeral 26 denotes an air/water feeding cylinder in which the air/water feeding button 13 is disposed. Reference numeral 27 denotes a suction cylinder in which the suction button 14 is disposed.

As shown in Fig. 4, the endoscope connector 30 is configured so that the first unit 31 and the second unit 32 are detachably attachable to each other.

The second unit 32 includes a second case body 32a and an exterior body 32b. The second case body 32a is, for example, made of metal and has electrical conductivity and rigidity. The second exterior body 32b is made of resin and has insulating properties. The second exterior body 32b is disposed so as to cover the outer circumferential face of the second case body 32a. A distal end side portion of the second case body 32a is configured so as to protrude by a predetermined amount from the distal end face of the second exterior body 32b. For example, two female screw portions 32f are provided at predetermined positions on a protruding portion of the second case body 32a.

As shown in Fig. 4 and Fig. 5, the first unit 31 includes a first case body 31 a and a first exterior body 31b. The first case body 31 a is, for example, made of metal and has electrical conductivity and rigidity. The first exterior body 31b is made of resin and has insulating properties. The first exterior body 31 b is disposed so as to cover the outer circumferential face of the first case body 31a in a manner such that the first exterior body 31b is movable in the axial direction with respect thereto. Two through-holes 31h are provided at predetermined positions in the proximal end side portion of the first case body 31a, that is, at positions corresponding to the female screw portions 32f. The aforementioned universal cable 7 is connected to the first case body 31a through a universal cable pipe sleeve portion 7b.

According to this configuration, a worker fits the second case body 32a that the second exterior body 32b covers inside the first case body 31a on which the first exterior body 31b is disposed, and aligns the positions of the female screw portions 32f with the positions of the through-holes 31h. Further, the worker screws unshown male screws into the female screw portions 32f through the through-holes 31h. As a result, the first case body 31a and the second case body 32a are integrally fixed to each other.

Thereafter, the worker performs an operation to fix the universal cable bend preventing element 42. That is, the worker performs an operation to attach the universal cable bend preventing element 42 to a predetermined position of the first case body 31 a on which the first exterior body 31b is disposed. At this time, the proximal end face of the bend preventing element 42 presses against the distal end face of the first exterior body 31 b. Thereupon, the first exterior body 31 b moves in the direction of the second exterior body 32b accompanying movement of the bend preventing element 42. As a result, the proximal end face of the first exterior body 31b is drawn near to and contacts against the distal end face of the second exterior body 32b. As the result of completing attachment of the universal cable bend preventing element 42, the proximal end face of the first exterior body 31 b and the distal end face of the second exterior body 32b are held in a watertight state, and the proximal end face of the universal cable bend preventing element 42 and the distal end face of the first exterior body 31b are held in a watertight state.

Note that a notch groove 31m and a cable release 31s are formed at predetermined positions in the side portion of the first case body 31a, as shown in Fig. 5. The notch groove 31 m and the cable release 31s constitute a cable extension port.

On the other hand, an extension hole (unshown) that corresponds to the notch groove 31m is formed in the side portion of the first exterior body 31b. As shown in Fig. 4, the extension hole is plugged so as to be held in a watertight state by attachment of the signal transmission cable bend preventing element 43 in a watertight state to the first exterior body 31b.

Next, operations of the endoscope 2 that includes the endoscope connector 30 having the above configuration will be described.

The endoscope connector 30 of the endoscope 2 is constituted by mounting the second unit 32 to the first unit 31 that is integrated with the proximal end portion of the universal cable 7. The connectors 51, 52, and 53 are fixedly installed at the distal end face of the second unit 32.

The notch groove 31m that is formed in the side portion of the first case body 31 a that constitutes the first unit 31 that is provided at the proximal end portion of the universal cable 7 has a notch portion 31c in a distal end side thereof in the axial direction.

According to this configuration, a worker can utilize the notch groove 31m and the cable release 31s to perform work to dispose the image pickup cable 21 and the signal lines 22, 23, and 24 inside the first case body 31a, or wiring routing work such as work to dispose the image pickup cable 21 and the signal lines 22, 23, and 24 on the outside. Examples of the wiring routing work include work to lead the image pickup cable 21 and the signal lines 22, 23, and 24 out from the notch groove 31m to the electrical connector 34, and work to store an excess length of the image pickup cable 21 or the like that has been generated by the leading out work by leading the excess length out from the cable release 31s and winding the excess length around a cable winding portion 31 u. As a result, routing of the image pickup cable 21 and the signal lines 22, 23, and 24 can be easily performed. Further, work to pull out the proximal end portions of the respective conduits 15, 16, and 17 to the proximal end side of the first unit 31 and work to attach the proximal end portions of the respective conduits 15, 16, and 17 to the connectors 51, 52, and 53 can be easily performed.

Work to integrally assemble the second unit 32 and the first unit 31 can be easily completed by performing an operation to connect the respective conduits 15, 16, and 17 and the connectors 51, 52, and 53, an operation to integrally fix the first case body 31a and the second case body 32a to each other with screws, and lastly an operation to mount the universal cable bend preventing element 42 at a predetermined position of the first unit 31.

The endoscope connector 30 is constituted by the first unit 31 and the second unit 32 in the above described manner. Consequently, in the assembly work, the respective conduits 15, 16, and 17 and the image pickup cable 21 and the signal lines 22, 23, and 24 can be reliably prevented from intertwining inside the endoscope connector 30. Further, the image pickup cable 21 and the signal lines 22, 23, and 24 can be reliably prevented from breaking during the assembly work. Accordingly, assembly workability can be enhanced and a burden of a worker can be reduced.

On the other hand, if a malfunction occurs in the endoscope 2 due to, for example, a blockage in the suction conduit 17 or the like, a worker detaches the second unit 32 from the first unit 31. The worker checks if there is a blockage in the in-unit suction conduit 59 inside the second unit 32, and also checks if there is a blockage in the suction conduit 17 that is extended from the first unit 31.

In this case, if the worker finds a blockage in only the in-unit suction conduit 59, the worker replaces the second unit 32 with a new second unit 32, and attaches the new second unit 32 to the first unit 31 to complete the repair operation. The worker disposes of the second unit 32 in which the blockage was found, or alternatively performs an operation to remove the blockage therefrom.

If the worker finds a blockage in the in-unit suction conduit 59 and the suction conduit 17, the worker performs an operation to replace the second unit 32 in which the blockage was found, and performs an operation to remove the blockage from the suction conduit 17.

In a case where the worker disposes of the second unit 32, the worker only needs to perform an operation to replace the second unit 32 with a new second unit 32 that is a new part, and therefore the time required for the repair can be shortened. On the other hand, if the worker performs an operation to remove the blockage from the second unit 32, since it is necessary to detach only the second unit 32 and perform an operation to remove the blockage therefrom, not only is the operation easy to perform, but the expenses involved in the repair can also be reduced since it is not necessary to buy a new part to repair the second unit 32.

By adopting a configuration in which the endoscope connector 30 is constituted by the first unit 31 and the second unit 32 in this manner, the need to perform work to remove the image pickup cable 21 and the signal lines 22, 23, and 24 from the electrical connector 34 and withdraw the image pickup cable 21 and the signal lines 22, 23, and 24 from the inside of the endoscope connector 30 one time is eliminated. In addition, since repair work can be performed after removing the second unit 32 from the universal cable 7 of the endoscope 2, the work can be easily performed, and work to check for a malfunction as well as repair and replacement work can be performed quickly.

Further, if a breakage occurs in the image pickup cable 21 or the signal lines 22, 23, and 24, the image pickup cable 21 or the signal lines 22, 23, and 24 can be replaced without removing the conduits 15, 16, and 17.

Specific procedures for replacing the signal lines 22, 23, and 24 will now be described.

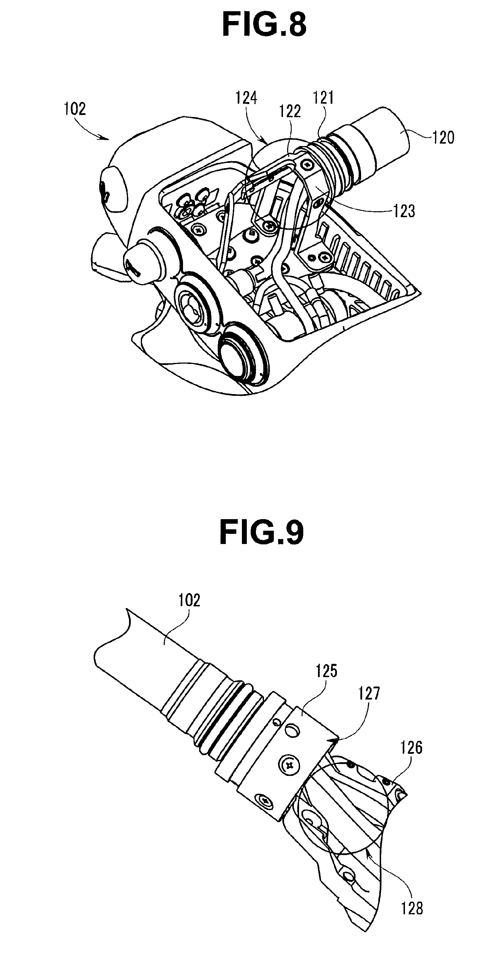

First, a worker opens the case of the electrical connector 34 and releases the connection with the substrate. Next, the worker connects new signal lines 22, 23, and 24 that are to be used as replacements to the proximal end portions of the signal lines 22, 23, and 24 that are to be replaced. Next, as shown in Fig. 8 that is described later, the worker opens the side portion of the operation portion 6 and disconnects the signal lines 22, 23, and 24 on this side. Thereafter, the worker draws out the signal lines 22, 23, and 24 from within the universal cable 7 and the signal transmission cable 33 from the opening portion shown in Fig. 8. Next, when the new signal lines 22, 23, and 24 that were connected to the proximal end portions of the broken signal lines 22, 23, and 24 are exposed, the worker connects the both end portions of the signal lines 22, 23, and 24, respectively, to complete the replacement operation.

Next, specific procedures for replacing the image pickup cable 21 will be described.

First, the worker releases the connection portion between the distal end portion 8 and the bending portion 9 shown in Fig. 1, and takes out the image pickup apparatus 20 therefrom. Next, the worker disconnects the image pickup cable 21 in the vicinity of the image pickup unit that includes the image pickup apparatus 20 and the image pickup cable 21. The worker then connects a rear end of a new image pickup cable 21 to the disconnected distal end of the image pickup cable 21. Next, the worker draws out the image pickup cable 21 from the electrical connector 34 side. When the new image pickup cable 21 that has been connected to the broken image pickup cable 21 is exposed, the worker connects each of the both end portions of the image pickup cable 21. Thereafter, the worker reconnects the distal end portion 8 and the bending portion 9 to complete the replacement operation.

As described above, even when it is necessary to replace or repair the electrical connector 34, it is possible to replace or repair the electrical connector 34 while the conduits 15, 16, and 17 remain connected thereto.

Furthermore, according to the present embodiment, an RFID chip is contained inside the convex portion with a built-in tag 44 of the first unit 31. It is therefore possible to easily check for an initial failure of the RFID chip during the assembly work, and in a case where the RFID chip is faulty, an operation to replace the RFID chip can be easily performed.

On the other hand, if a malfunction has occurred in the RFID chip that is incorporated into the endoscope 2, the following procedures are performed to replace the RFID chip.

First, the worker detaches the second unit 32 from the first unit 31 as described above. Next, the worker replaces the RFID chip that is disposed inside the first unit 31 with a new RFID chip. In this case, if the RFID chip is embedded in the first exterior body 31b, the worker replaces the first exterior body 31b together with the RFID chip. Thus, similarly to the above described case, work to replace the RFID chip can be performed without the need to perform an operation to withdraw the image pickup cable 21 and the signal lines 22, 23, and 24 one time from inside the endoscope connector 30.

In the above described embodiment a configuration is adopted in which the earth terminal 41 is provided in the first unit 31. However, a configuration may also be adopted in which the earth terminal 41 is provided in the second unit 32. In this case, a configuration is adopted in which, in addition to placing the earth terminal 41 in a conductive state with respect to the second case body 32a, unshown screws that are used to integrally fix the first case body 31 a and the second case body 32a to each other are members with electrical conductivity.

In this connection, an operation to check for and identify a location at which watertightness has been breached in an endoscope can be performed by feeding air into the inner space of the endoscope through a water leakage detecting pipe sleeve. However, even when a location at which watertightness is breached has been identified, it has been difficult to identify how far moisture that entered from the breach location reached within the inner space of the endoscope.

Accordingly, for example, in an endoscope in which watertightness was breached in the vicinity of a joint between a universal cable and an operation portion, it is difficult to determine whether or not water that entered from the breach location reached the inside of an endoscope connector. Therefore, for example, in the case of a configuration that includes a substrate or the like inside the endoscope connector, conventionally the substrate has been replaced regardless of whether or not the substrate was exposed to water.

Consequently, there has been a need for technology that, upon identifying a location at which watertightness was breached, can identify which sites within the inner space of the endoscope have been reached by water that entered from the location at which the watertightness was breached.

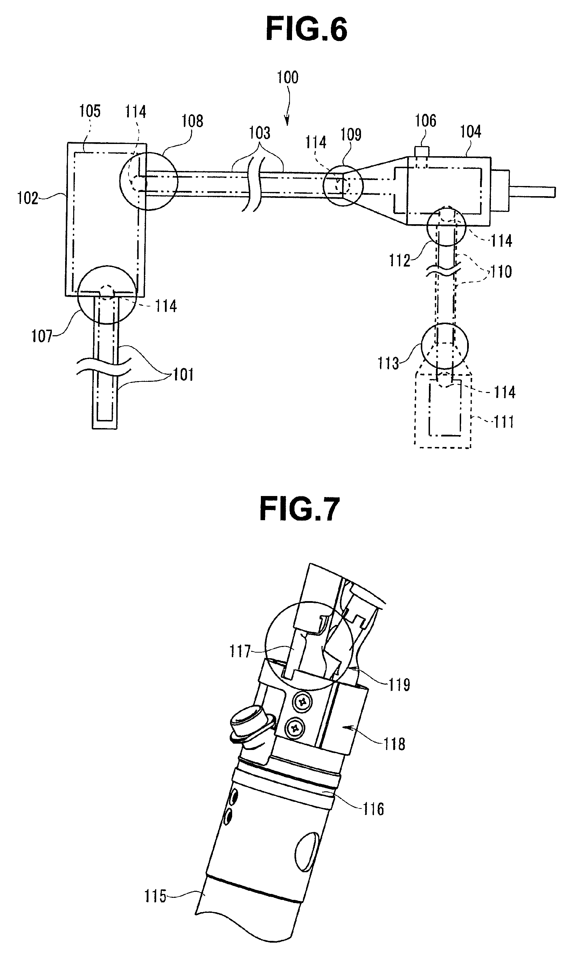

As shown in Fig. 6, in an endoscope 100, an insertion portion 101, an operation portion 102, and a universal cable 103 are connected in series. An endoscope connector 104 is provided at a proximal end portion of the universal cable 103. A water leakage detecting pipe sleeve 106 that includes a check valve is provided in the endoscope connector 104. The water leakage detecting pipe sleeve 106 communicates with an endoscope inner space 105 that is shown by a chain double-dashed line in Fig. 6.

The endoscope 100 of the present embodiment includes a first connection site 107 that connects the insertion portion 101 and the operation portion 102, a second connection site 108 that connects the operation portion 102 and the universal cable 103, and a third connection site 109 that connects the universal cable 103 and the endoscope connector 104.

Further, depending on the kind of endoscope, a signal transmission cable 110 may extend from a side portion of the endoscope connector 104, as indicated by a dashed line in Fig. 6. An electrical connector 111 is provided at an end portion of the signal transmission cable 110.

The endoscope 100 configured in this manner also includes a fourth connection site 112 and a fifth connection site 113 in addition to the aforementioned connection sites. The fourth connection site 112 connects the endoscope connector 104 and the signal transmission cable 110. The fifth connection site 113 connects the signal transmission cable 110 and the electrical connector 111.

In the present embodiment, for example, water immersion detecting seals 114 as water leakage detecting members are provided in the internal space of the connection sites 107, 108 109, 112, and 113, respectively. Each of the water immersion detecting seals 114 is, for example, a seal with a polka-dot pattern, in which the polka-dot pattern becomes blurred if moisture comes in contact therewith.

Specifically, at the first connection site 107, the water immersion detecting seal 114 is attached at a first region 119 in the vicinity of a joining portion 118 at which, as shown in Fig. 7, an insertion portion pipe sleeve 116 and an operation portion framework part 117 are integrally joined and fixed together. The insertion portion pipe sleeve 116 is provided at a proximal end of an insertion portion constituent member 115 that is included in the insertion portion 101. The operation portion framework part 117 constitutes an insertion portion side of the operation portion 102.

Further, at the second connection site 108, the water immersion detecting seal 114 is attached at a second region 124 in the vicinity of a joining portion 123 at which, as shown in Fig. 8, a first universal pipe sleeve 121 and an operation portion framework part 122 are integrally joined and fixed together. The first universal pipe sleeve 121 is provided at one end of a cable constituent member 120 that is included in the universal cable 103. The operation portion framework part 122 constitutes a universal cable side of the operation portion 102.

Further, at the third connection site 109, the water immersion detecting seal 114 is attached at a third region 128 in the vicinity of a joining portion 127 at which, as shown in Fig. 9, a second universal pipe sleeve 125 and a connector framework part 126 are integrally joined and fixed together. The second universal pipe sleeve 125 is provided at the other end of the cable constituent member 120 that is included in the universal cable 103. The connector framework part 126 constitutes a universal cable side of the endoscope connector 104.

Accordingly, for example, in a case where a worker identified a breach in the watertightness of the insertion portion 101, if the water immersion detecting seal 114 of the first connection site 107 has reacted, the worker can determine that moisture has entered the inside of the operation portion 102. In addition, if the water immersion detecting seal 114 of the second connection site 108 has not reacted, the worker can determine that moisture did not reach the inside of the universal cable 103.

The water immersion detecting seals 114 are provided in this manner at the connection sites 107, 108, 109, 112, and 113, respectively. According to this configuration, when a worker has identified a location at which watertightness has been breached, the worker can easily and reliably determine which sites have been reached by moisture that entered from the identified location at which the watertightness was breached by checking the reaction states of the water immersion detecting seals 114.

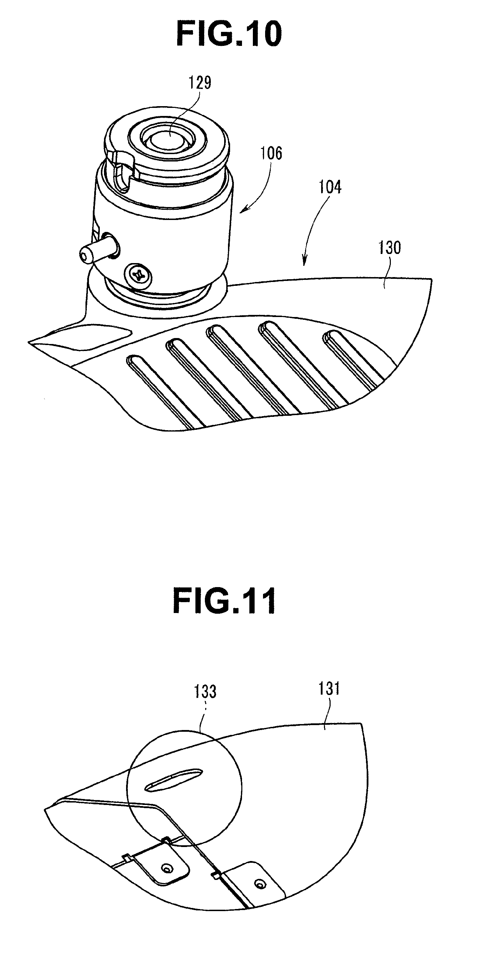

Note that when the water leakage detecting pipe sleeve 106 is provided in the endoscope 100, there is a risk of water entering when a check valve 129 shown in Fig. 10 is temporarily opened. Consequently, in the endoscope connector 104 to which the water leakage detecting pipe sleeve 106 can be mounted, an unshown water immersion detecting seal is attached at a pipe sleeve inner face region 133 that is adjacent to the water leakage detecting pipe sleeve of a metal frame 131 that also serves as a shield member that is shown in Fig. 11. The pipe sleeve inner face region 133 is disposed on an inner side of a connector exterior member 130.

As a result, in the event that moisture infiltrates towards the endoscope inner space 105 of the endoscope connector 104 through the water leakage detecting pipe sleeve 106, the water immersion detecting seal of the pipe sleeve inner face region 133 will react and the worker can thus determine that moisture has infiltrated into the endoscope connector 104.

In the above described embodiment the endoscope is configured to include a water leakage detecting pipe sleeve. However, a configuration may also be adopted in which a water immersion detecting seal is provided at each connection site of an endoscope that does not include a water leakage detecting pipe sleeve. According to this configuration, whether or nor the watertightness has been breached can be checked when repairing the endoscope, and a countermeasure can be taken at the time of repair.

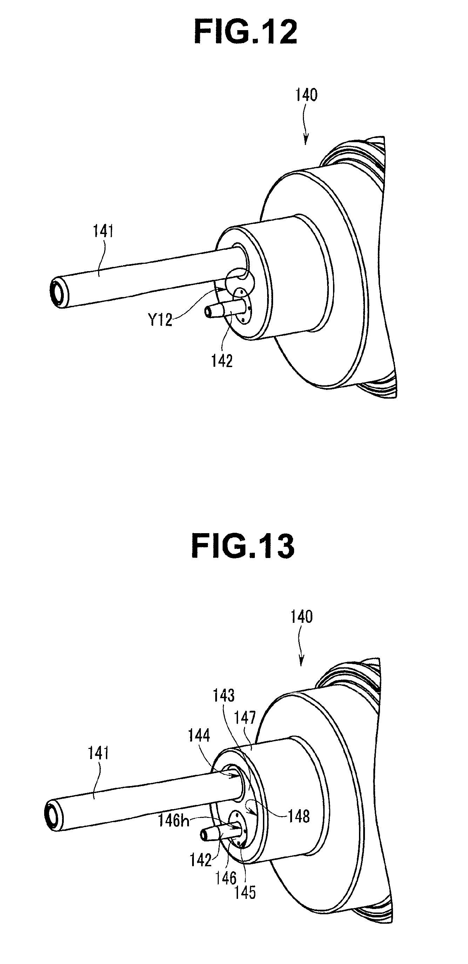

In this connection, various kinds of pipe sleeves are disposed in an endoscope connector. Further, accompanying the miniaturization of endoscope connectors, there is a tendency for pipe sleeves to be disposed adjacent to each other. For example, as shown in Fig. 12, in some cases a light guide pipe sleeve 141 and an air feeding pipe sleeve 142 are disposed adjacent to each other in an endoscope connector 140. In this configuration, the wall thickness of the connector is thin between the light guide pipe sleeve 141 and the air feeding pipe sleeve 142, as indicated by an arrow Y12. When the exterior of the endoscope connector 140 is made of resin, the rigidity at the thin-walled portion decreases and the chemical resistance thereof also decreases and thus the thin-walled portion is a fragile portion. There is a risk that the fragile portion will be a cause of degradation over time in the endoscope connector 140.

Therefore, with respect to an endoscope connector that includes a plurality of pipe sleeves, there has been a desire for a configuration in which a fragile portion is no longer present and the strength of the endoscope connector has been reinforced.

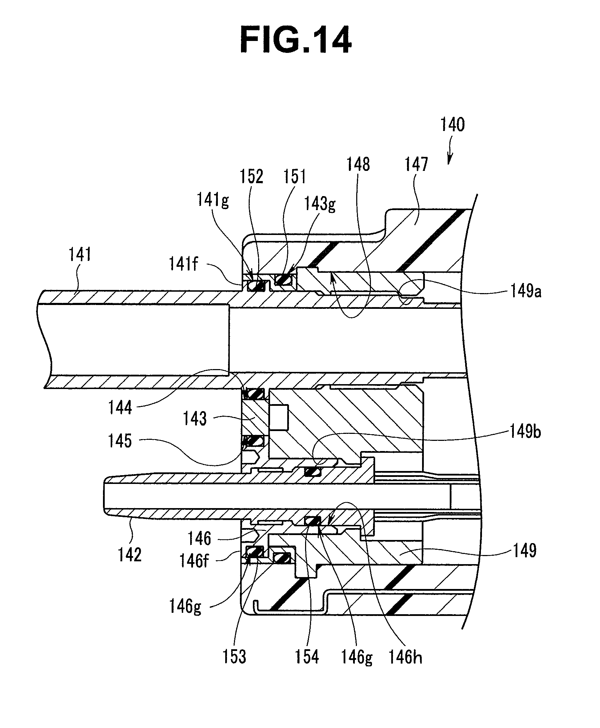

As shown in Fig. 13 and Fig. 14, the endoscope connector 140, for example, includes a pipe sleeve holding member 143 in which the light guide pipe sleeve 141 and the air feeding pipe sleeve 142 are arranged. The pipe sleeve holding member 143 is a disk member made of, for example, stainless steel or the like that has a higher degree of strength than resin. The pipe sleeve holding member 143 includes a light guide pipe sleeve insertion hole 144 and an air feeding pipe sleeve mounting hole 145.

Note that a wall thickness between the light guide pipe sleeve insertion hole 144 and the air feeding pipe sleeve mounting hole 145, a wall thickness from the outer circumference of the pipe sleeve holding member 143 to the light guide pipe sleeve insertion hole 144, and a wall thickness from the outer circumference of the pipe sleeve holding member 143 to the air feeding pipe sleeve mounting hole 145 are designed so as to have a predetermined rigidity.

The light guide pipe sleeve 141 is disposed in the light guide pipe sleeve insertion hole 144 of the pipe sleeve holding member 143 and thus provided in the pipe sleeve holding member 143. The air feeding pipe sleeve 142 is disposed in an air feeding pipe sleeve disposing hole 146h of an air feeding pipe sleeve mounting member 146, and in addition, the air feeding pipe sleeve mounting member 146 is disposed in the air feeding pipe sleeve mounting hole 145 of the pipe sleeve holding member 143 and thus provided in the pipe sleeve holding member 143. The pipe sleeve holding member 143 in which the light guide pipe sleeve 141 and the air feeding pipe sleeve 142 are provided is disposed inside a holding hole 148. In this disposition state, the light guide pipe sleeve 141 and the air feeding pipe sleeve 142 are arranged in the endoscope connector 140 in a manner in which the light guide pipe sleeve 141 and the air feeding pipe sleeve 142 protrude to the outside therefrom. The holding hole 148 is a through-hole that communicates the inner space of the connector that is formed in an endoscope connector proximal end convex portion 147 made of resin that is an exterior member with outside.

Reference numeral 151 denotes a first O-ring. The first O-ring 151 is disposed in a pipe sleeve circumferential groove 143g formed in an outer circumferential face of the pipe sleeve holding member 143. The first O-ring 151 maintains watertightness between the holding hole 148 and the pipe sleeve holding member 143.

Reference numeral 152 denotes a second O-ring. The second O-ring 152 is disposed in a flange circumferential groove 141 g formed in an outer circumferential face of a light guide flange 141 f. The light guide flange 141 f is formed in the outer circumference of the light guide pipe sleeve 141.

The second O-ring 152 maintains watertightness between the light guide pipe sleeve insertion hole 144 and the light guide pipe sleeve 141. Note that the light guide flange 141 f is a holding member that holds the pipe sleeve holding member 143 so that the pipe sleeve holding member 143 does not drop from the holding hole 148.

Reference numeral 153 denotes a third O-ring. The third O-ring 153 is disposed in a flange circumferential groove 146g formed in an outer circumferential face of a mounting member flange 146f. The mounting member flange 146f is formed in the outer circumference of an end portion of the air feeding pipe sleeve mounting member 146. The third O-ring 153 maintains watertightness between the air feeding pipe sleeve mounting hole 145 and the air feeding pipe sleeve mounting member 146. Note that the mounting member flange 146f is a holding member that holds the pipe sleeve holding member 143 so that the pipe sleeve holding member 143 does not drop from the holding hole 148.

Reference numeral 154 denotes a fourth O-ring. The fourth O-ring 154 is disposed in an air feeding pipe sleeve circumferential groove 142g formed in the outer circumference of an end portion of the air feeding pipe sleeve 142. The fourth O-ring 154 maintains watertightness between the air feeding pipe sleeve disposing hole 146h and the air feeding pipe sleeve 142.

Reference numeral 149 denotes a proximal end convex portion constituent member. The proximal end convex portion constituent member 149 is fixed at a predetermined position of the holding hole 148. The proximal end convex portion constituent member 149 is, for example, made of metal, and includes a light guide pipe sleeve fixing installation hole 149a and an air feeding pipe sleeve fixing installation hole 149b that are through-holes in the axial direction. In the pipe sleeve holding member 143, the light guide pipe sleeve 141 is fixedly installed in the light guide pipe sleeve fixing installation hole 149a and the air feeding pipe sleeve 142 is fixedly installed in the air feeding pipe sleeve fixing installation hole 149b. Thus, the configuration is one in which light guide pipe sleeve 141 and the air feeding pipe sleeve 142 are disposed in a manner in which the light guide pipe sleeve 141 and the air feeding pipe sleeve 142 do not fall from the holding hole 148.

Thus, in the case of a configuration in which a plurality of pipe sleeves are adjacently disposed in an endoscope connector made of resin, a pipe sleeve holding member that is made of metal and that has a plurality of holes corresponding to the pipe sleeves is arranged in a holding hole of the endoscope connector. As a result, an endoscope connector can be realized in which a thin-walled portion that is made of resin does not exist between the pipe sleeves.

Note that although two pipe sleeves, namely the light guide pipe sleeve 141 and the air feeding pipe sleeve 142, are provided in the above described embodiment, the pipe sleeves according to the present invention are not limited to the aforementioned two kinds, i.e. the light guide pipe sleeve 141 and the air feeding pipe sleeve 142, and may be other pipe sleeves. Further, the number of holes formed in the pipe sleeve holding member 143 is not limited to two, and may be more than two. In addition, the pipe sleeve holding member 143 is not limited to a disk member, and may be an elliptic member or a rectangular member or the like.

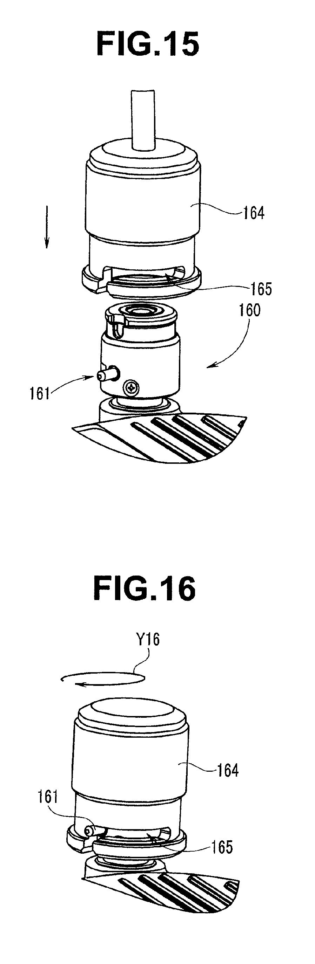

In this connection, a water leakage detecting pipe sleeve, for example, is provided in an endoscope connector for the purpose of detecting the watertightness of an endoscope. As shown in Fig. 15, a cam pin 161 constituted by a locking screw is provided in a water leakage detecting pipe sleeve 160.

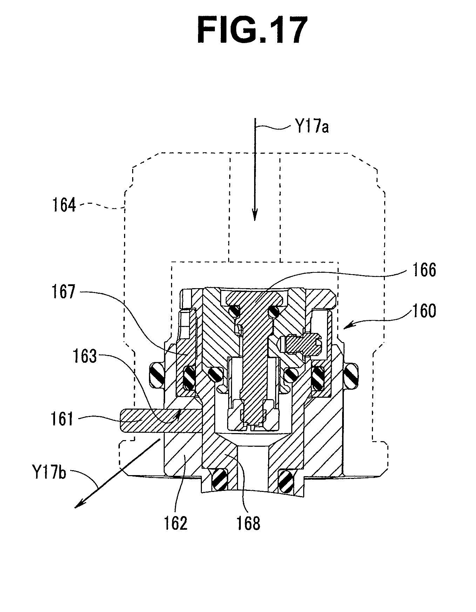

As shown in Fig. 17, the cam pin 161 is mounted by, for example, screwing in a locking screw mounting hole 163 that is a through-hole formed in a side portion of a pipe sleeve body 162.

As shown in Fig. 15, an air pipe sleeve 164 that supplies air can be mounted to the water leakage detecting pipe sleeve 160. A cam groove 165 of a predetermined shape is provided in the air pipe sleeve 164.

When mounting the air pipe sleeve 164 to the water leakage detecting pipe sleeve 160, a worker disposes the cam pin 161 inside the cam groove 165 as shown in Fig. 16. The worker rotates the air pipe sleeve 164 along the cam groove 165 as indicated by an arrow Y16. In this case, a structure is adopted in which the water leakage detecting pipe sleeve 160 is opened accompanying rotation of the air pipe sleeve 164.

In this operational state, a large load acts on the cam pin 161 in the rotational direction and a check valve movement direction. This load is a factor that generates looseness at the screwing portion between the cam pin 161 and the locking screw mounting hole 163.

When looseness is generated at the screwing portion by the load, backlash occurs at the cam pin 161. As a result, air that is supplied through the air pipe sleeve 164 as indicated by an arrow Y17a in Fig. 17 enters through a gap between the pipe sleeve body 162 and an annular member 167. Thereafter, the air passes through a gap between the pipe sleeve body 162 and a sliding member 168 and a gap that is caused by play between the cam pin 161 and the locking screw mounting hole 163 as an air path, and leaks to outside as indicated by an arrow Y17b. As a result, there has been a risk of a malfunction occurring in which air of a desired pressure can not be supplied quickly into the inner space of the endoscope. Further, there has also been a risk of a path being formed through which moisture will enter the inner space of the endoscope from a gap between the cam pin 161 and the locking screw mounting hole 163.

Consequently, with respect to the water leakage detecting pipe sleeve 160, there has been a need for a configuration that prevents backlash occurring at the cam pin 161, a configuration that can reliably supply air of a desired pressure into the inner space of the endoscope even if backlash has occurred, and a configuration in which entry of moisture into the inner space of the endoscope is reliably prevented.

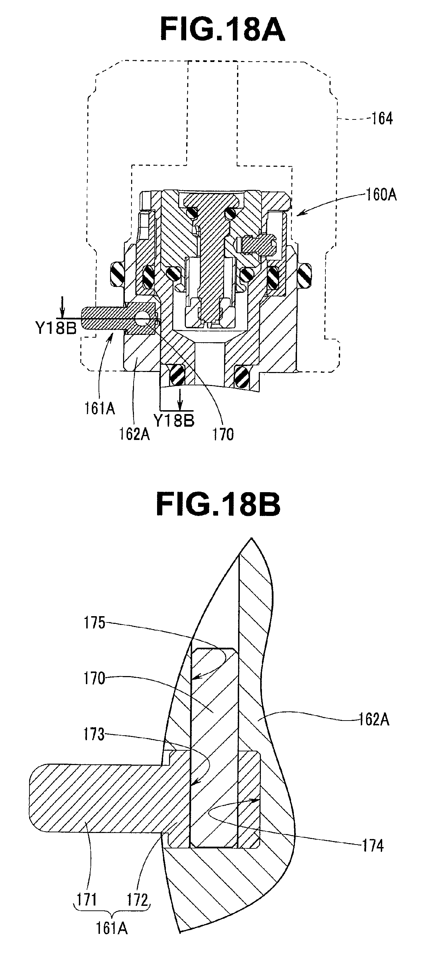

A water leakage detecting pipe sleeve 160A shown in Figs. 18A and 18B has a configuration in which, instead of mounting the cam pin 161 in the locking screw mounting hole 163 by screwing, the cam pin 161A is fixed by a locking pin 170.

The cam pin 161A includes a cam portion 171 and a fixing portion 172. The cam portion 171 is disposed in the cam groove 165. In the present embodiment, the fixing portion 172 has a larger diameter than the cam portion 171. A pin engagement hole 173 that is a through-hole in which the locking pin 170 is engageably inserted is formed in the fixing portion 172.

On the other hand, a fixing portion disposing hole 174 and a communicating hole 175 are formed in the pipe sleeve body 162A. The fixing portion disposing hole 174 has a bottom face on which the fixing portion 172 is disposed. The communicating hole 175 communicates the fixing portion disposing hole 174 with outside. The fixing portion disposing hole 174 is designed so as to have a predetermined depth dimension towards the center of the pipe sleeve body 162A from a side. The communicating hole 175 is configured to have a central axis that is orthogonal to a central axis of the fixing portion disposing hole 174 from the side portion.

Procedures for fixing the cam pin 161A in the pipe sleeve body 162A will now be described.

First, a worker disposes the fixing portion 172 of the cam pin 161A so as to contact against the bottom face of the fixing portion disposing hole 174. At this time, the worker makes the orientation of the pin engagement hole 173 of the fixing portion 172 orthogonal to the longitudinal axis direction of the water leakage detecting pipe sleeve 160A. As a result, the communicating hole 175 that communicates with the fixing portion disposing hole 174 is disposed on substantially the same axis as the pin engagement hole 173.

Next, the worker inserts the locking pin 170 into the communicating hole 175 and disposes the locking pin 170 inside the pin engagement hole 173 so that an end face of the locking pin 170 enters a predetermined state with respect to the communicating hole 175. Thereafter, for example, the worker applies adhesive to the opening side of the fixing portion disposing hole 174. As a result, the cam pin 161 A and the pipe sleeve body 162A are integrally fixed to each other.

After disposing the fixing portion 172 of the cam pin 161A in the bottomed fixing portion disposing hole 174 in this manner, the worker carries out positioning of the locking pin 170 that is inserted from the side portion of the pipe sleeve body 162A into the fixing portion 172. As a result, even if the fixing portion 172 of the cam pin 161A is in a loose state with respect to the fixing portion disposing hole 174, the fixing portion disposing hole 174 that is a bottomed hole is prevented from serving as an air path. Accordingly, it is possible to reliably prevent a situation in which supplied air leaks to outside, and also a situation in which a path is formed through which moisture enters the inner space of the endoscope.

In the above described embodiment, a configuration is adopted in which the cam pin 161A is integrally fixed to the pipe sleeve body 162A by means of the locking pin 170. However, a configuration may also be adopted in which the cam pin 161A is integrally fixed by screwing to the pipe sleeve body 162A using the aforementioned locking screw instead of the locking pin 170. In this configuration, a process is performed to form a female screw in the communicating hole 175 and the pin engagement hole 173.

In this connection, one end side of a universal cable that is included in an endoscope is attached to an operation portion, and the other end side thereof is attached to an endoscope connector. Consequently, a cable pipe sleeve is fixedly installed on one end side and on the other end side of a cable constituent member that is included in the universal cable. The pipe sleeves that are disposed at the both ends are fixed so as to be in a predetermined positional relationship with respect to a circumferential direction of the cable constituent member. Therefore, a worker performs the fixing operation while performing alignment in the rotational direction of the pipe sleeves. Specifically, first, the worker applies adhesive to the pipe sleeves. Next, the worker mounts the pipe sleeves on which the adhesive is applied to the cable constituent member. Subsequently, the worker rotates the pipe sleeves to adjust the rotational positions thereof. However, because adhesive has been applied to the pipe sleeves, rotation of the pipe sleeves causes air bubbles to enter the adhesive, and there is a risk of the air bubbles becoming a cause of a watertightness failure.

Therefore, there has been a need for a universal cable with which positioning in the rotational direction of the two pipe sleeves can be easily performed, and which is equipped with pipe sleeves that have a configuration that can reliably prevent formation of air bubbles that cause a watertightness failure at both ends of a cable constituent member.

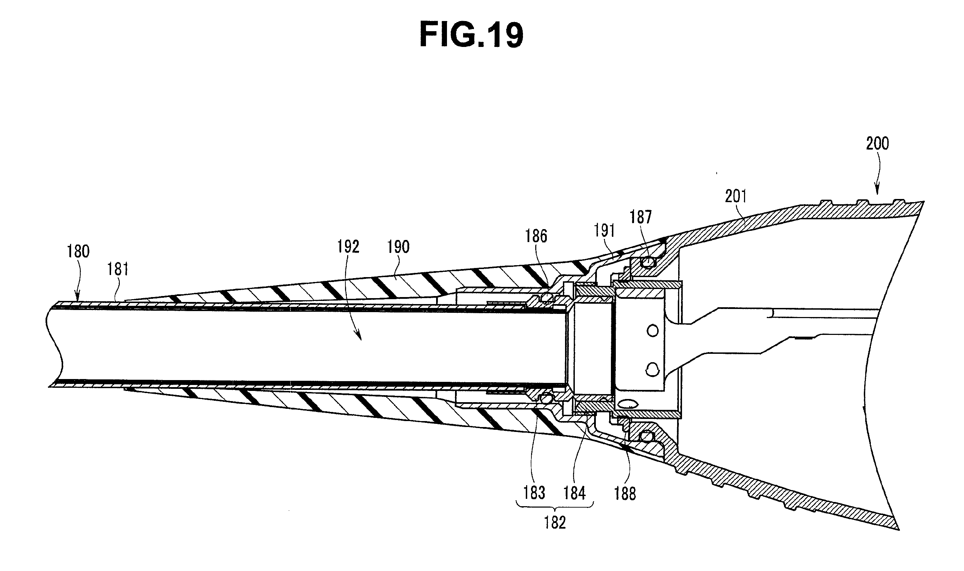

The configuration of a universal cable will now be described referring to Fig. 19 to Fig. 23.

Note that the configurations of the both end portions of the universal cable of the present embodiment are identical. Therefore, the configuration of an end portion on one side is described hereinafter, and a description of the configuration of the other end portion is omitted.

As shown in Fig. 19, one end portion of a universal cable 180 is arranged so as to be fixed to an endoscope connector 200 that is a portion to be connected. Reference numeral 190 denotes a bend preventing element that, for example, is made of synthetic rubber and has a predetermined resilience, and protects a connection portion of the universal cable 180. Reference numeral 191 denotes a bend preventing element insert member (hereunder, abbreviated as "insert member") that is made of metal and is formed in a predetermined shape.

Note that the other end of the universal cable 180 is arranged so as to be fixed to an operation portion (not shown) that is a portion to be connected.

A cable pipe sleeve 182 is provided at the end portion of a cable constituent member 181 included in the universal cable 180. The cable pipe sleeve 182 includes a first pipe sleeve 183 and a second pipe sleeve 184.

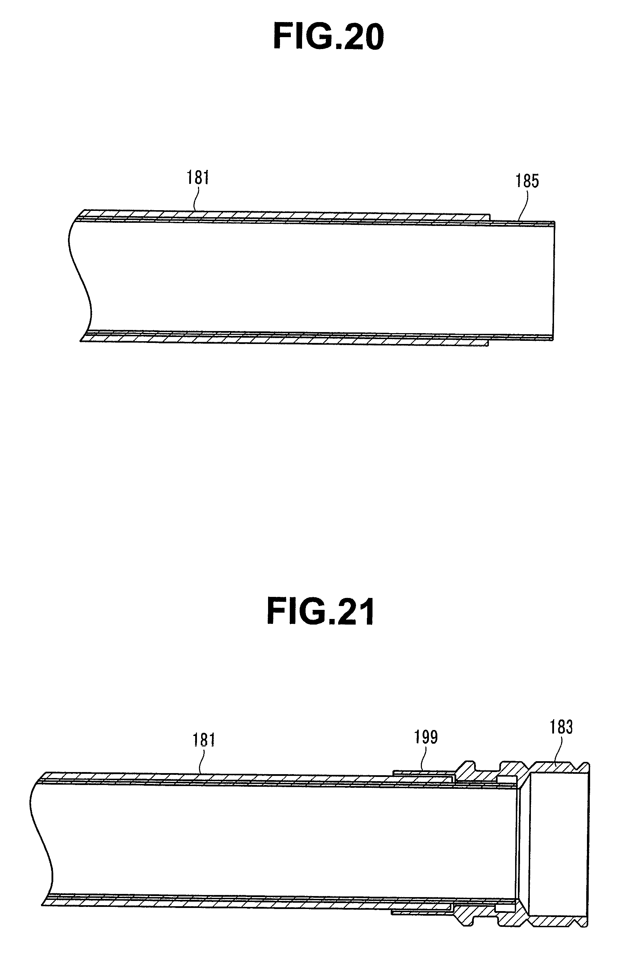

The first pipe sleeve 183 is fixed to the end portion of the cable constituent member 181. Specifically, the first pipe sleeve 183 is disposed on a cable flexible portion 185 that is provided in an exposing manner at an end portion of the cable constituent member 181 as shown in Fig. 20. As shown in Fig. 21, an adhesive 199 is applied onto the first pipe sleeve 183, and the first pipe sleeve 183 is adhesively fixed to the cable flexible portion 185 in a manner that ensures watertightness. In other words, the first pipe sleeve 183 is fixed to the cable constituent member 181 without defining the positioning thereof in the circumferential direction. Note that the cable flexible portion 185 is exposed by a predetermined amount from the cable end face.

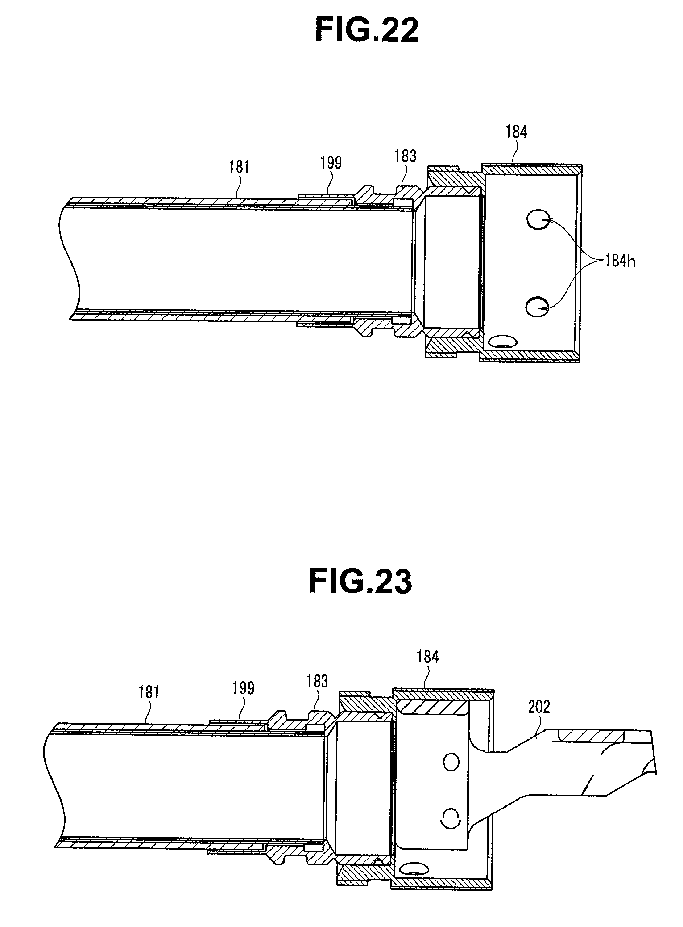

As shown in Fig. 19 and Fig. 22, the second pipe sleeve 184 is adhesively fixed by an unshown adhesive to the end portion of the first pipe sleeve 183. As shown in Fig. 22, two screw holes 184h are provided at predetermined positions in the second pipe sleeve 184. Therefore, in a state in which adhesive has been applied thereto, the second pipe sleeve 184 is mounted to an end portion of the first pipe sleeve 183, and thereafter the second pipe sleeve 184 is rotated to adjust the rotational position thereof with respect to the circumferential direction of the cable constituent member 181 (positional adjustment with respect to a cable pipe sleeve that is fixed at the opposite side) and fixed. As a result, the two screw holes 184h of the second pipe sleeve 184 are defined at predetermined positions.

Note that a connector framework part 202 is integrally fixed to the inner surface of the second pipe sleeve 184 by fastening with screws as shown in Fig. 23.

As shown in Fig. 19, a first O-ring 186 is provided on an outer circumferential face of the first pipe sleeve 183. The first O-ring 186 intimately contacts the inner face of the bend preventing element insert member 191. A connector exterior member 201 is disposed on the outer circumferential face on the proximal end side of the second pipe sleeve 184. A second O-ring 187 is provided on the outer circumferential face of the distal end side of the connector exterior member 201. The second O-ring 187 intimately contacts the inner face of the bend preventing element insert member 191. The distal end face of the connector exterior member 201 is disposed in contact with a fixing ring 188. The fixing ring 188 is disposed on the outer circumferential face on the proximal end side of the second pipe sleeve 184.

The endoscope connector 200 is disposed at one end portion of the universal cable 180. In the endoscope 200 to which the bend preventing element 190 is integrally fixed, the first O-ring 186 and the second O-ring 187 intimately contact with the respective inner faces of the insert member 191 so that watertightness is ensured. The first pipe sleeve 183 is adhesively fixed to the cable flexible portion 185 in a manner that ensures watertightness.

Accordingly, a space between the first O-ring 186 and the second O-ring 187 is configured as an endoscope inner space 192 that is constituted by the inner space of the universal cable 180 and the inner space of the endoscope connector 200 and the like. An adhesive portion between the first pipe sleeve 183 and the second pipe sleeve 184, that is, an adhesive portion for which a watertightness ensuring function is not required, is disposed inside the endoscope inner space 192.

By providing the cable pipe sleeve 182 that is constituted by the first pipe sleeve 183 and the second pipe sleeve 184 at both end portions of the universal cable in this manner, adhesive fixing for ensuring watertightness and adhesive fixing for defining rotational positions can be performed by respectively different processes.

Further, by adopting a configuration in which an adhesive portion between the first pipe sleeve 183 and the second pipe sleeve 184 is disposed within the endoscope inner space 192, a watertightness ensuring function is not required with respect to the adhesive portion between the first pipe sleeve 183 and the second pipe sleeve 184.

Consequently, adhesive fixing of two kinds of pipe sleeves can be easily performed, definition of rotational positions can be reliably performed, adhesive portions can be prevented from becoming the cause of a watertightness failure, and the workability can be improved.

It should be understood that the present invention is not limited only to the above described embodiments, and various modifications can be made without departing from the spirit or scope of the invention.

The present application is filed claiming the priority of Japanese Patent Application No. 2012-018783 filed in Japan on January 31, 2012, and the above described disclosure is incorporated by reference in the present description, claims and drawings.

## Claims

1. An endoscope comprising an endoscope connector at a proximal end portion of a universal cable that is extended from an endoscope operation portion,
wherein the endoscope connector comprises:
a first unit that is provided at the proximal end portion of the universal cable, and inside which are inserted a fluid conduit and a signal transmission line that are inserted through inside of the universal cable;
a second unit that is connected and fixed to the first unit, and that internally comprises a connection conduit that is connected to the fluid conduit; and
a signal transmission cable that is integrated with a side portion of the first unit and includes an electrical connector at an end portion.

2. The endoscope according to claim 1, wherein the first unit and the second unit are detachably attachable to each other.

3. The endoscope according to claim 2, wherein the second unit comprises:
a connection portion that is detachably connectable on a connecting portion side with the first unit, and to which one end of the connection conduit is fixed; and
an external connection portion to which the other end of the connection conduit is fixed at least at one of a side portion that is different from the connecting portion side and an end portion that faces the connecting portion.

4. The endoscope according to claim 2, wherein a solid body identification portion is provided inside the first case body.
